**Europäisches Patentamt**

**European Patent Office**

**Office européen des brevets**

(19)

(11) Publication number: **0 198 880**
**B1**

# EUROPEAN PATENT SPECIFICATION

(12)

(45) Date of publication of the patent specification:
**31.05.89**

(21) Application number: **85905244.1**

(22) Date of filing: **17.10.85**

(86) International application number:
**PCT/SE 85/00399**

(87) International publication number:
**WO 86/02634 (09.05.86 Gazette 86/10)**

(51) Int. Cl.⁴: **C 07 C 37/00,** C 07 C 39/00,
C 07 C 39/02, C 07 C 45/61,
C 07 C 47/00

(54) **A METHOD FOR DEALKYLATION OF ALKYL-ARYL ETHERS.**

(30) Priority: **22.10.84 SE 8405262**

(43) Date of publication of application:
**29.10.86 Bulletin 86/44**

(45) Publication of the grant of the patent:
**31.05.89 Bulletin 89/22**

(84) Designated Contracting States:
**CH DE FR GB LI**

(56) References cited·
**DE-B-1 768 050**
**GB-A-2 013 653**
**NO-C-107 714**
**US-A-4 087 410**
**US-A-4 172 960**

**Tetrahedron Letters, vol. 25, no. 32, 1984, M.
Vovekananda Bhatt and J. Ramesh Babu, "New
reagents 3: Aluminium iodide - a highly
regioselective ether - cleaving reagent with novel
cleavage pattern", pp. 3497-3500**
**Chemical Abstracts, vol. 78 (1973), abstract no.
71603v, Ann.Chim. (Rome), 1972, 62 (7-8) 505-12 (Ital.)**
**Chemical Abstracts, vol. 83 (1975), abstract no.
36919j, J. Inorg. Nucl. Chem. 1975, 37 (1), 293-5 (Eng.)**
**Chemical Abstracts, vol. 73 (1970), abstract no.
24547s, Ric. Sci. 1969, 39 (4-6), 424-7 (Ital.)**

(73) Proprietor: **CHEMICAL DYNAMICS SWEDEN AB,
Box 212, S-532 00 Skara (SE)**

(72) Inventor: **ANDERSSON, Sven, Göran, Bertil,
Disponentgatan 21:231, S-211 57 Malmö (SE)**

(74) Representative: **Lautmann, Kurt O., KURT
LAUTMANNS PATENTBYRA AB Box 245, S-691 25
Karlskoga (SE)**

**Description**

The present invention relates to a method for dealkylation of alkyl-aryl ethers with aluminium triiodide as defined in the claims.

Dealkylation of ethers is a well known metod in organic syntheses and many reagents can be used. Earlier methods have however considerable disadvantages when used on a technical scale. Hydrogen iodide, hydrogen bromide and hydrogen chloride as well as aluminium trichloride and ferrichloride give bad yields. Aluminium tribromide gives better yield but is an expensive raw material. Good yields can be obtained with boron trichloride, boron tribromide and boron triiodide but these reagents are very expensive and the methods are only of interest for laboratory work. Some phosphorus compounds can be used but are not very selective.

The use of aluminium triiodide for dealkylation has earlier been tested. It is known that $AlJ_3$ is a highly reactive aromatic-aliphatic ether cleaving reagent and it is also known that $AlJ_3$-$PhOCH_3$ complexes have an increased tendency to undergo ether cleavage, compared to their Cl- and Br-analogues. The cleavage of alkoxybenzenes with $AlJ_3$ is also described.

According to an earlier method aluminium metal and iodine reacted in carbon disulphide and the ether was then added in a carbon disulphide solution. The disadvantage with this method is the use of the toxic and inflammable carbon disulphide and the rather long reaction times. Byproducts are formed, which in some cases have very bad smell and involve industrial hygiene problems.

Cleavage of the two methyl groups of 1-(tertiary alkyl)-3,5-dimethoxy-benzene by heating with pyridine hydrochloride is known as well as the cleavage of an ether with the chloroiodate of a quaternary ammonium tensioactive compound.

The present method has important advantages as to industrial hygiene and gives a pure product and high yields. The reaction times are also short. The method is characterized by the use of inert solvents which are inexpensive and relatively easy to handle and by the use of aluminium triodide, prepared in situ from iodine and aluminium powder, conducting the dealkylation reaction in the presence of a catalytic amount of a quaternary ammonium iodide of the general formula $R_4N^+I^-$, where R is an alkyl group, in an inert aliphatic, cycloaliphatic or aromatic solvent.

The reaction can be expressed as follows:

$$R - O - Ar + AlI_3 \xrightarrow[\text{2) H}_2\text{O}]{\text{1) R}_4\text{NI}} Ar - OH + R - I$$

R = alkyl
Ar = aryl

The method will be further illustrated with the following examples:

**Example 1**

Aluminium powder (2,5 g, 93 mmol) and iodine (19,0 g, 150 mmol) were mixed in 130 ml benzene (or cyclohexane) and were refluxed until the red colour of iodine disappeared (about 1,5 hours). The mixture was cooled. A solution of anisol (phenyl - methyl ether, 5,4 g, 50 mmol) and n-Bu$_4$N$^+$I$^-$ (tetra-n-butyl ammoniumiodine, 0,05 g, 0,14 mmol) dissolved in 25 ml benzen (cyclohexane) was added dropwise. The mixture was heated and was refluxed for 20 min, then cooled and hydrolysed with 150 ml water. The organic phase was separated and the water phase was extracted with 2 x 25 ml diethyl ether. The organic phases were collected and were extracted with 30 ml 2 molar NaOH. The water phase was separated, acidified with concentrated HCl and was extracted with 3 x 25 ml diethyl ether. The organic phases were dried (Na$_2$SO$_4$) and were evaporated to give pure crystalline phenol. Yield = 99 %. M.p. 41 - 42°C.

**Example 2**

Aluminium powder (75 g, 279 mmol) and iodine (57 g, 450 mmol) were mixed and refluxed as in example 1. After cooling solution of pyrogalloltrimethylether, 1,2,3-trimethoxibenzene (8,4 g, 50 mmol ) and n-Bu$_4$N$^-$I$^-$ (0,1 g, 0,28 mmol) in 25 ml benzene was added. After refluxing for 0,5 hours the mixture was hydrolyzed with 150 ml water. The benzene phase was separated and washed with 2 x 20 ml water, which was added to the water phase.

The combined water phases were extracted with 6 x 50 ml ethylacetate (EtOAc). The EtOAc-phases were combined, dried (Na$_2$SO$_4$) and evaporated to give pure pyrogallol in a 95 % yield. M.p. 132 - 133°C.

The following table gives a survey of other dealkylations made according to the method.

| Ether | Solvents | Molar ratios | | Reaction time (h) | Yield % | Product |
|---|---|---|---|---|---|---|
| | | AlI₃/ether | ether/R₄NI· | | | |
| anisol | benzene | 1 | 360 | 1/3 | 100 | phenol |
| anisol | cyclo-hexane | 1 | 360 | 1/3 | 100 | phenol |
| anisol | CS₂ (for comparison) | 1 | 360 | 1/3 | 91 | phenol |
| 2-methoxy-naphthalene | benzene | 1 | 360 | 1/3 | 94 | 2-hydroxy-naphthlene |
| p-Methoxyben-zaldehyde | benzene | 1 | 360 | 1/2 | 78 | p-Hydroxy-benzaldehyde |
| | cyclo-hexane | 1 | 360 | 1/2 | 80 | p-Hydroxy-benzaldehyde |
| m-methoxyben-zaldehyde | benzene | 1 | 360 | 1/2 | 84 | m-Hydroxy-benzaldehyde |
| pyrogallol-trimethylether | benzene | 3 | 180 | 1/2 | 95 | pyrogallol |
| vanilline | benzene | 4.8 | 360 | 1.5 | 88 | 2.3-Dihydroxy-benzaldehyde |

| Ether | Solvents | Molar ratios | | Reaction time (h) | Yield % | Product |
|---|---|---|---|---|---|---|
| | | AlI₃/ether | ether/R₄NI⁻ | | | |

| Ether | Solvents | AlI₃/ether | ether/R₄NI⁻ | Reaction time (h) | Yield % | Product |
|---|---|---|---|---|---|---|
| Diphenylether (for comparison) | benzene | 1 | 360 | 1 | trace | phenol |
| ethoxybenzene | cyclohexane | 1 | 360 | 1.0 | 90 | phenol |
| 4-Hydroxy-3-methoxytoluene | cyclohexane | 2 | 100 | 0.7 | 98 | 3.4-Dihydroxytoluene |
| isovanilline | cyclohexane | 5.5 | 120 | 2.5 | 14 | 3.4-Dihydroxybenzaldehyd = protokotechualdehyde |
| 3.4-mehtylendioxybenzaldehyde = piperonal = heliotropin | cyclohexane | 4.5 | 120 | 1.5 | 84 | |
| 2,4,5-trimethoxybenzaldehyde | cyclohexane " " " | 1.5 3.3 5.5 10.0 | 100 100 100 50 | 14 1.5 1.5 10.0 | 88 90 91 85 | 4.5-Dimethoxysalicylaldehyde |
| 3,4,5-trimethoxybenzaldehyde | cyclohexane " " | 1.5 3.3 5.5 | | 12.0 6.0 15.0 | 1) 7(1) 41(2) 16(1) 84(2) 10(1) 81(2) | 4,5-Dimethoxy-2-hydroxybenzaldehyde |

4

| Ether | Solvents | Molar ratios | | Reaction | Yield | Product |
|---|---|---|---|---|---|---|
| | | $AlI_3$/ether | ether/$R_4NI^-$ | time (h) | % | |

Molar ratio ether/$R_4NI^-$ was 100 in all these experiments with 3,4,5-trimethoxybenzaldehyde

3,5-Dimethoxy-4-hydroxybenzaldehyde

2/

3,4-Dihydroxy-5-methoxybenzaldehyde

3,5-Dihydroxy-4-methoxybenzaldehyde

## Claims

1. A process for the dealkylation of an alkyl aryl ether by reaction with aluminium triiodide, prepared in situ from iodine and aluminium powder, characterized in conducting said reaction in the presence of a catalytic amount of a quaternary ammonium iodide of the general formula $R_4N^+I^-$, where R is an alkyl group, in an inert aliphatic, cycloaliphatic or aromatic solvent.

2. Method according to claim 1, characterized in that the quaternary ammonium iodide is tetra-n-butylammonium iodide.

3. Method according to claim 1 or 2, characterized in that the solvent is cyclohexane.

4. Method according to claim 1, characterized in that the solvent is benzene.

5. Method according to claim 1, chararacterized in that the solvent is toluene.

6. Method according to claim 1, characterized in that the solvent is xylene.

## Patentansprüche

1. Verfahren für Dealkylierung von Alkylaryläthern durch Reaktion mit Aluminiumtrijodid, in situ aus Jod und Aluminiumpulver hergestellt, dadurch gekennzeichnet dass die Reaktion im Gegenwart von einer katalytischen Menge von einem quartären Ammoniumjodid der allgemeinen Formel $R_4N^+J^-$, wo R eine Alkylgruppe ist in einem inerten aliphatischen, cycloaliphatischen oder aromatischen Lösungsmittel durchgeführt wird.

2. Verfahren laut Anspruch 1, dadurch gekennzeichnet dass das quartäre Ammoniumjodid Tetra-n-butylammoniumjodid ist.

3. Verfahren laut Anspruch 1 oder 2, dadurch gekennzeichnet dass das Lösungsmittel Cyklohexan ist.

4. Verfahren laut Anspruch 1, dadurch gekennzeichnet dass, das Lösungsmittel Bensol ist.

5. Verfahren laut Anspruch 1, dadurch gekennzeichnet dass, das Lösungsmittel Toluol ist.

6. Verfahren laut Anspruch 1, dadurch gekennzeichnet dass, das Lösungsmittel Xylol ist.

**Revendications**

1. Procédé de désalkylation d'un éther alkyle aryle par réaction avec le triodure d'aluminium préparé in situ à partir d'iode et de poudre d'aluminium, caractérisé en ce que ladite réaction s'effectue en présence d'une quantité catalytique d'un iodure d'ammonium quaternaire de formule générale $R_4N^+I^-$, où R est un groupe alkyle, dans un solvant inerte aliphatique, cycloaliphatique ou aromatique.

2. Procédé selon la revendication 1, caractérisé en ce que l'iodure d'ammonium quaternaire est le tetra-n-iodure de butylammonium.

3. Procédé selon la revendication 1 ou 2, caractérisé en ce que le solvant est le cyclohexane.

4. Procédé selon la revendication 1, caractérisé en ce que le solvant est le benzène.

5. Procédé selon la revendication 1, caractérisé en ce que le solvant est le toluène.

6. Procédé selon la revendication 1, caractérisé en ce que le solvant est le xylène.